(19) Europäisches Patentamt / European Patent Office / Office européen des brevets

(11) **EP 3 687 448 B1**

(12) **EUROPEAN PATENT SPECIFICATION**

(45) Date of publication and mention
of the grant of the patent:
**26.03.2025 Bulletin 2025/13**

(21) Application number: **18842744.7**

(22) Date of filing: **20.12.2018**

(51) International Patent Classification (IPC):
**A61F 2/16** *(2006.01)*

(52) Cooperative Patent Classification (CPC):
**A61F 2/1613;** A61F 2002/1696; A61F 2230/0004

(86) International application number:
**PCT/IB2018/060467**

(87) International publication number:
**WO 2019/123390 (27.06.2019 Gazette 2019/26)**

(54) **INTRAOCULAR LENSES HAVING AN ANTERIOR-BIASED OPTICAL DESIGN**

INTRAOKULARLINSEN MIT ANTERIOR-VORGESPANNTEM OPTISCHEM DESIGN

LENTILLES INTRAOCULAIRES PRÉSENTANT UNE CONCEPTION OPTIQUE À SOLLICITATION ANTÉRIEURE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **20.12.2017 US 201762608037 P**

(43) Date of publication of application:
**05.08.2020 Bulletin 2020/32**

(73) Proprietor: **Alcon Inc.
1701 Fribourg (CH)**

(72) Inventors:
• **CAMPIN, John Alfred
Fort Worth, Texas 76134 (US)**
• **CHOI, Myoung-Taek
Fort Worth, Texas 76134 (US)**
• **CURATU, Costin Eugene
Fort Worth, Texas 76134 (US)**
• **PETTIT, George Hunter
Fort Worth, Texas 76134 (US)**
• **WEI, Xin
Frisco, Texas 75035 (US)**

(74) Representative: **Murgitroyd & Company
165-169 Scotland Street
Glasgow G5 8PL (GB)**

(56) References cited:
US-A1- 2005 203 619    US-A1- 2006 227 286
US-A1- 2007 093 891    US-A1- 2015 320 547
US-A1- 2017 112 612

**Description**

**FIELD**

The present disclosure relates generally ophthalmic lenses and, more particularly, to intraocular lenses having an anterior-biased optical design.

**BACKGROUND**

The human eye in its simplest terms functions to provide vision by transmitting light through a clear outer portion called the cornea, and focusing the image by way of a lens onto a retina. The quality of the focused image depends on many factors including the size and shape of the eye, and the transparency of the cornea and lens. When age or disease causes the lens to become less transparent, vision deteriorates because of the diminished light which can be transmitted to the retina. This deficiency in the lens of the eye is medically known as a cataract. An accepted treatment for this condition is surgical removal of the lens and replacement of the lens with an intraocular lens (IOL)

An IOL typically includes (1) an optic that corrects the patient's vision (e.g., typically via refraction or diffraction), and (2) haptics that constitute support structures that hold the optic in place within the patient's eye (e.g., within capsular bag). In general, a physician selects an IOL for which the optic has the appropriate corrective characteristics for the patient. During the surgical procedure, the surgeon may implant the selected IOL by making an incision in the capsular bag of the patient's eye (a capsulorhexis) and inserting the IOL through the incision. Typically, the IOL is folded for insertion into the capsular bag via a corneal incision and unfolded once in place within the capsular bag. During unfolding, the haptics may expand such that a portion of each contacts the capsular bag, retaining the IOL in place.

Although existing IOLs may function acceptably well in many patients, they also have certain shortcomings. For example, existing IOL may have bi-convex optical designs and may be formed of a material having a refractive index that necessitates anterior surface curvatures in a range known to cause reflections. This phenomenon is sometimes referred to a "glint" or "scary eye."

Accordingly, what is needed is an IOL having an optical design that anterior surface curvatures that reduce the incidence of glint across a large range of powers.

Reference is made to US2015/320547, US 2017/112612, US2007/093891 and US 2006/227286 which have been cited as relating to the state of the art. US2007/093891 for example relates to an intraocular lens having an optical power and a shape factor for implantation in an eye of a patient in which the natural lens has been removed

**SUMMARY**

It will be appreciated that the scope is in accordance with the claims. Accordingly, there is provided a computer implemented method of manufacturing an intraocular lens as claimed in the independent claim. Further features are provided in the dependent claims. The specification may include further arrangements outside the scope of the claims provided as background and to assist in understanding the invention.

In certain arrangements of the specification, an ophthalmic lens includes an optic having an anterior surface with an anterior surface radius of curvature ($R_1$) and a posterior surface with a posterior surface radius of curvature ($R_2$). The anterior surface radius of curvature ($R_1$) and the posterior surface radius of curvature ($R_2$) define a shape factor (X) (where $X = (R_2-R_1)/(R_2+R_1)$) that is greater than zero. The shape factor (X) corresponds to a curve defining shape factor (X) as a function of lens power (P), the curve monotonically decreasing with increased lens power (P).

In certain embodiments, the present disclosure may provide one or more technical advantages. As one example, IOLs having the above-described anterior-biased optical design may reduce the prevalence of "glint," a phenomenon in which an external observer sees a reflection from an IOL implanted in a patient's eye. Human eye model simulations have shown that the intensity of the reflection depends on the anterior surface radius of curvature of the IOL, the intensity of the reflection being strongest in a certain range of radii of curvature that more closely match the curvature of the wavefront incident on the IOL (e.g., radii of curvature in the range of 18-40 mm). IOLs having the above-described anterior-biased optical design may result in anterior surface radii of curvature across a broad range of IOL powers that are outside the range known to cause the highest intensity reflections, thereby reducing the prevalence of glint.

As another example, IOLs having the above-described anterior-biased optical design may result in a stable effective lens position (ELP) across a broad range of IOL powers due to the fact that the distance between the IOL principal plane and the IOL haptic plane remains substantially constant across the power range. This stability of ELP across the IOL power range may minimize A-constant variation, which may result in better and/or more predictable refractive outcomes.

As yet another example, IOLs having the above-described anterior-biased optical design may be less sensitive to misalignment (e.g., decentration and tilt). More particularly, the above described IOLs have a positive shape factor, meaning they have a relatively high anterior surface curvature. The relatively high anterior surface curvature means that light rays incident on and outgoing from the anterior surface have a small average angle from normal directions such that the Gaussian optics formula deviates from Snell's law by a small amount, decreasing sensitivity to decentration. As a result, IOLs having the above-described anterior-biased optical design may result in better refractive outcomes.

As a final example, IOLs having the above-described anterior-biased optical design may reduce the incidence of negative dysphotopsia post-implantation. One reason for the reduction in the incidence of negative dysphotopsia is that the amount of posterior shift of the iris after cataract surgery may be reduced for IOLs having the above-described anterior-biased optical design. Because it is hypothesized that posterior iris shift may result in the patient experiencing negative dysphotopsia due to light hitting or missing different parts of the retina, reducing such shift may reduce the incidence of negative dysphotopsia. Another reason for the reduction in the incidence of negative dysphotopsia is that peripheral rays (rays entering the patient eye at a high incidence angle) that hit an IOL having the above-described anterior-biased optical design may be spread more evenly as compared to IOLs having an equi-convex design (i.e., same anterior and posterior curvatures). This even spreading of peripheral rays may reduce the perception of negative dysphotopsia.

## BRIEF DESCRIPTION OF THE DRAWINGS

For a more complete understanding of the present disclosure and the advantages thereof, reference is now made to the following description taken in conjunction with the accompanying drawings in which like reference numerals indicate like features and wherein:

FIG. 1 illustrates a top view of an exemplary ophthalmic lens, according to certain embodiments of the present disclosure;

FIG. 2 illustrates a cross-sectional view of the optic of the exemplary ophthalmic lens depicted in FIG. 1 (along line A-A of FIG. 1);

FIG. 3 illustrates a plot of shape factor (X) versus lens power (P) for an exemplary ophthalmic lens;

FIG. 4 illustrates a plot of anterior surface radius of curvature ($R_1$) and posterior surface radius of curvature ($R_2$) versus lens power (P) for an exemplary ophthalmic lens;

FIG. 5 illustrates a plot of anterior surface power ($P_1$) and posterior surface power ($P_2$) versus lens power (P) for an exemplary ophthalmic lens;

FIG. 6 illustrates a plot of the distance between lens haptic plane and lens principle plane versus lens power (P) for an exemplary ophthalmic lens; and

FIG. 7 illustrates a plot of lens power shift versus lens power (P) for an exemplary ophthalmic lens.

The skilled person in the art will understand that the drawings, described below, are for illustration purposes only. The drawings are not intended to limit the scope of the applicant's disclosure in any way.

## DETAILED DESCRIPTION

In general, the present disclosure relates to an IOL having an anterior-biased optical design, the IOL including an optic having an anterior surface with an anterior surface radius of curvature ($R_1$) and a posterior surface with a posterior surface radius of curvature ($R_2$). The anterior surface radius of curvature ($R_1$) and the posterior surface radius of curvature ($R_2$) define a shape factor (X) (where $X = (R_2-R_1)/(R_2+R_1)$) that is greater than zero. The shape factor (X) corresponds to a curve defining shape factor (X) as a function of lens power (P), the curve monotonically decreasing with increased lens power (P).

An IOL having the above-described anterior-biased optical design may reduce the prevalence of glint by maintaining anterior curvatures across a broad range of IOL powers that are outside a range of curvatures known to cause reflections. Additionally, an IOL having the above-described anterior-biased optical design may result in better and/or more predictable refractive outcomes by (1) providing a substantially constant distance between the IOL principal plane and IOL haptic plane across a broad range of IOL powers, thereby providing stable ELP and reduced A-constant variation across the power range, and (2) reducing sensitivity to misalignment (e.g., decentration and tilt).

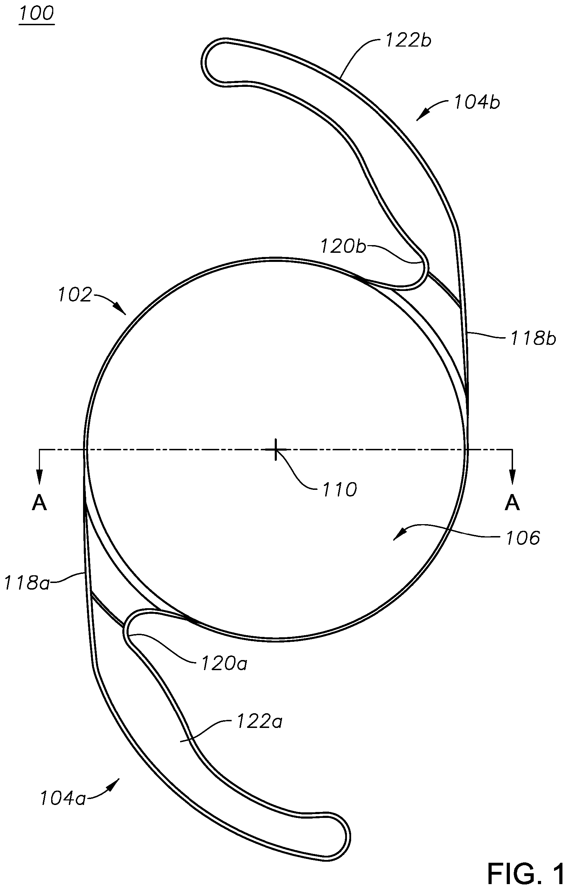

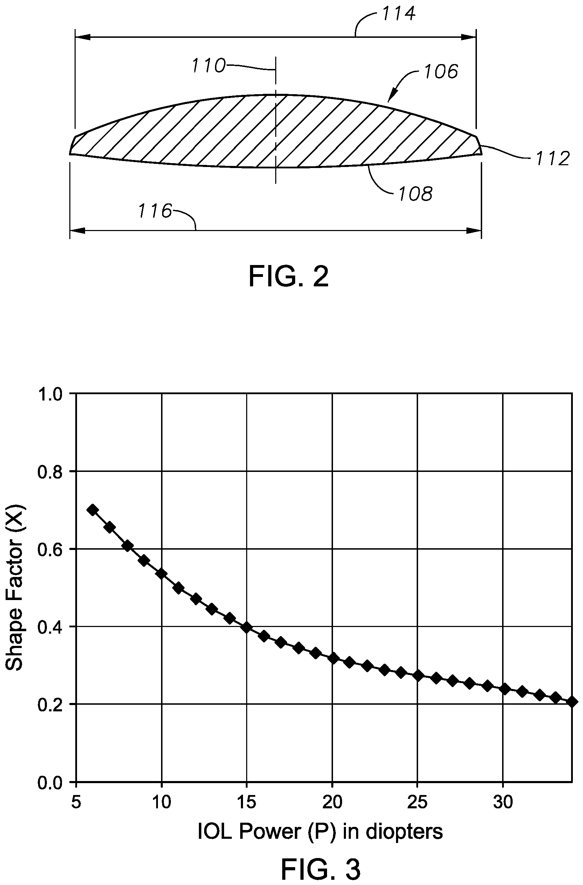

FIGS. 1-2 illustrate an exemplary ophthalmic lens 100 (referred to below as IOL 100) having an optic 102 and a plurality of haptics 104. In particular, FIG. 1 illustrates a top view of IOL 100 and FIG. 2 illustrates a cross-sectional view of the optic 102 of the IOL 100 (along line A-A of FIG. 1).

A variety of techniques and materials can be employed to fabricate the IOL 100. For example, the optic 102 of an IOL 100 can be formed of a variety of biocompatible polymeric materials. Some suitable biocompatible materials include, without limitation, soft acrylic polymeric materials, hydrogel materials, polymethymethacrylate, or polysulfone, or polystyrene-containing copolymeric materials, or other biocompatible materials. By way of example, in one embodiment, the optic 102 may be formed of a soft acrylic hydrophobic copolymer such as those described in U.S. Patent Nos. 5,290,892; 5,693,095; 8,449,610; or 8,969,429. The haptics 104 of the IOL 100 can also be formed of suitable biocompatible materials, such as those discussed above. While in some cases, the optic 102 and haptics 104 of an IOL can be fabricated as an integral unit, in other cases they can be formed separately and joined together utilizing techniques known in the art.

Optic 102 may include an anterior surface 106, a posterior surface 108, an optical axis 110, and an optic edge 112. Anterior surface 106 and/or posterior surface 108 may include any suitable surface profiles for correcting a patient's vision. For example, anterior surface 106 and/or posterior surface 108 may be spheric, aspheric, toric, refractive, diffractive, or any suitable combination thereof. In other words, optic 102 may be one or more of a spheric lens, an aspheric lens, a toric lens, a multifocal lens (refractive or diffractive), an extended depth of focus lens, any suitable combination of the foregoing, or any other suitable type of lens.

Anterior surface 106 may have an anterior surface diameter 114 between 4.5 mm and 7.0 mm. In one specific embodiment, anterior surface diameter 114 may be approximately 6 mm. Additionally, anterior surface 106 may comprise a full surface optic, meaning that the optic portion of anterior surface 106 extends to the optic edge 112. Alternatively, anterior surface 106 may include one or more transition regions (not depicted) between an edge of the optic region of anterior surface 106 and the optic edge 112.

Posterior surface 108 may have a posterior surface diameter 116 between 4.5 mm and 7.0 mm. In one specific embodiment, posterior surface diameter 116 may be approximately 6.15 mm (or may vary, depending on lens power, within a range including 6.15 mm). Additionally, posterior surface 108 may comprise a full surface optic, meaning that the optic portion of posterior surface 108 extends to the optic edge 112. Alternatively, posterior surface 108 may include one or more transition regions (not depicted) between an edge of the optic region of posterior surface 108 and the optic edge 112.

Optic edge 112 may extend between anterior surface 106 and posterior surface 108 and may comprise one or more curved surfaces, one or more flat surfaces, or any suitable combination thereof. In one specific embodiment, optic edge 112 may comprise a continuously curved surface extending between anterior surface 106 and posterior surface 108. In such embodiments, the continuously curved surface may not include any tangents parallel to optical axis 110, which may advantageously reduce the incidence of positive dysphotopsia results, at least in part, from edge glare.

In certain embodiments, the thickness at optic edge 112 may be constant across the entire IOL power range (e.g., 6-35 diopters). As a result, a center thickness of IOL 100 (i.e., the thickness along optical axis 110) may vary across the IOL power range. As one example, the thickness at optic edge 112 may be approximately 0.25mm across the entire IOL power range.

Haptics 104 may each include a gusset region 118, an elbow region 120, and a distal region 122. Gusset region 118 may extend from the periphery of the optic 102 and may span an angle of the periphery of optic 102 (e.g. an angle greater than or equal to 50 degrees). Elbow region 120 may couple gusset region 118 and distal region 122 and may comprise a portion of the haptic 104 having the minimum width (e.g., between 0.40 mm and 0.65 mm. As a result, elbow region 128 may create a hinge allowing haptic 104 to flex while minimizing buckling and vaulting of optic 102. Distal region 130 may extend from elbow region 128 and may have a length in the range of 6 mm to 7.5mm. Although a particular number of haptics 104 having a particular configuration are depicted and described, the present disclosure contemplates that IOL 100 may include any suitable number of haptics 104 having any suitable configuration.

As defined in the claims, anterior surface 106 of optic 102 has an anterior surface radius of curvature ($R_1$) and posterior surface 108 of optic 102 has a posterior surface radius of curvature ($R_2$). Additionally, the anterior surface radius of curvature ($R_1$) and the posterior surface radius of curvature ($R_2$) may collectively define a shape factor (X) for the optic 102 as follows:

$$X = \frac{R_2 - R_1}{R_2 + R_1} \qquad\qquad \text{Eq. (1)}$$

In addition, anterior surface 106 has an anterior surface power ($P_1$) and posterior surface 108 has a posterior surface power ($P_2$), the anterior and posterior surface powers defined as follows:

$$P_1 = \frac{n_2 - n_1}{R_1} \qquad\qquad \text{Eq. (2)}$$

wherein,

$n_1$ is a refractive index of aqueous humor or a patient's eye (approximately 1.336);

$n_2$ is a refractive index of the optic 102;

The refractive index of the optic ($n_2$) may be in a range of 1.42 to 1.7. In certain embodiments, the refractive index of the optic ($n_2$) may be in a range of 1.42 to 1.56. In certain embodiments, the refractive index of the optic ($n_2$) may be approximately 1.498.

The shape factor (X) of the optic 102 is greater than zero, meaning that the posterior surface radius of curvature ($R_2$) is greater than the anterior surface radius of curvature ($R_2$) (i.e., the anterior surface curvature is greater than the posterior surface curvature). In certain embodiments, the shape factor (X) falls in the range of 0.20 to 1.0 for IOLs 100 having lens powers (P) in the range of 6 to 35 diopters.

As defined in the claims, the shape factor (X) corresponds to a curve defining shape factor (X) as a function of lens power (P), the curve monotonically decreasing with increased lens power (P). Due to manufacturing constraints or other manufacturing considerations, the shape factor (X) may not be equal to the value defined by the curve for a given lens power (P). The shape factor (X), however, may nevertheless be selected to correspond to the curve. For example, the shape factor (X) may correspond to the curve in that, for any given lens power (P), the shape factor (X) does not deviate from the curve by more than 0.2.

As defined in the claims, the above-described curve to which the shape factor (X) corresponds in non-linear. For example, the curve may be defined by the following cubic equation:

$$X = X_0 + X_1P + X_2P^2 + X_3P^3 \qquad \text{Eq. (4)}$$

wherein $X_0$, $X_1$, $X_2$, and $X_3$ are constants having values that are real numbers.

In certain embodiments, $X_0$ is in the range of 0.75 to 1.5, $X_1$ is a negative value in the range of -0.11 to -0.05, $X_2$ is in the range of 0.0017 to 0.0035, and $X_3$ is in the range of -.0.000042 to 0.00002. In certain embodiments, $X_0$ is approximately 1.068, $X_1$ is approximately -0.075, $X_2$ is approximately 0.0025, and $X_3$ is approximately -0.00003.

Given equations (1) - (4), the anterior surface radius of curvature ($R_1$), the posterior surface radius of curvature ($R_2$), the anterior surface power ($P_1$), and the posterior surface power ($P_2$) may be defined as follows:

$$R_1 = \frac{1}{\frac{1}{R_2} + \frac{P}{n_2 - n_1}} \qquad \text{Eq. (5)}$$

$$R_2 = \frac{2(n_2 - n_1)}{P(X - 1)} \qquad \text{Eq. (6)}$$

$$P_1 = \frac{n_2 - n_1}{R_1} = \frac{P(X + 1)}{2} \qquad \text{Eq. (7)}$$

$$P_2 = \frac{n_1 - n_2}{R_2} = \frac{P(1 - X)}{2} = P - P_1 \qquad \text{Eq. (8)}$$

In embodiments in which the shape factor (X) corresponds to a curve defined by Eq. (4) and $X_0$ is approximately 1.068, $X_1$ is approximately -0.075, $X_2$ is approximately 0.0025, and $X_3$ is approximately -0.00003, the curve to which the shape factor (X) corresponds is depicted in FIG. 3. As noted above, due to manufacturing constraints or other manufacturing considerations, the shape factor (X) may not be equal to the value defined by the depicted curve for all lens powers (P) but may nevertheless be selected to correspond to the curve (e.g., the shape factor (X) may not deviate from the curve by more than 0.2).

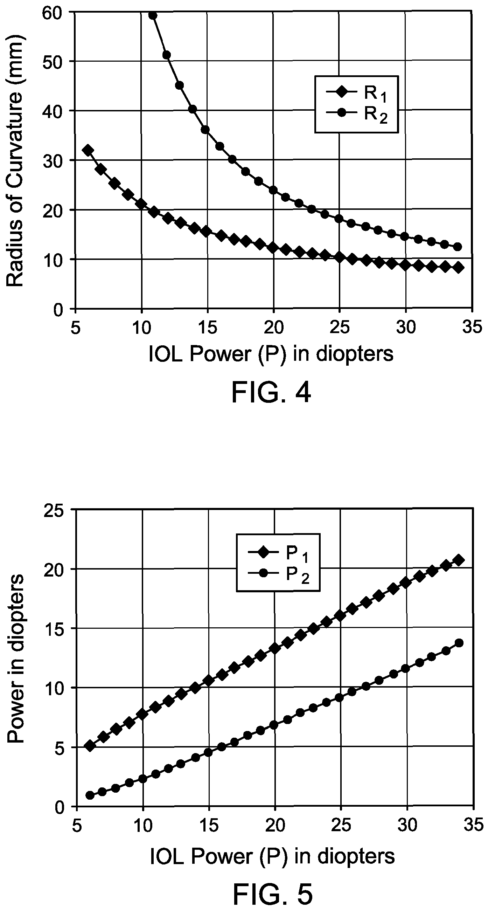

In embodiments in which optic 102 of IOL 100 has a refractive index of approximately 1.498 and the shape factor (X) corresponds to the curve depicted in FIG. 3, the radius of curvature of the anterior surface ($R_1$) and the radius of curvature of the posterior surface ($R_2$) may correspond to the curves depicted in FIG. 4. In such embodiments, the anterior surface power ($P_1$) and the posterior surface power ($P_2$) may correspond to the curves depicted in FIG. 5. Like the variation in shape factor (X) relative to the desired curve discussed above with regard to FIG. 3, manufacturing constraints or other manufacturing considerations may necessitate that, for a given lens power (P), the surface radii and/or powers may not be equal to the curves depicted in FIGS. 4-5. The surface radii and/or powers, like the shape factor (X), may nevertheless correspond to the curves in that they do not deviate from the curves by more than a certain amount (e.g., the anterior surface radius of curvature ($R_1$) may not deviate from the curve by more than 2mm).

In certain embodiments, one or both of the anterior surface 106 and the posterior surface may be aspheric. For example, anterior surface 106 may be aspheric, with the deviation from the base curvature (i.e., the above-described curvature of anterior surface 106) defined as follows:

$$sag = \frac{cr^2}{1+\sqrt{1-(1+k)c^2r^2}} + a_4r^4 + a^6r^6 \qquad \text{Eq. (9)}$$

wherein,

r denotes a radial distance from the optical axis 110;
c denotes a base curvature of the anterior surface 106;
k denotes a conic constant;
$a_4$ is a fourth order deformation constant;
$a_6$ is a sixth order deformation constant.

In certain embodiments, the constants of Eq. (9) ($k$, $a_4$, and $a_6$) may be selected such that a target spherical aberration for IOL 100 is achieved. As one example, the constants of Eq. (9) ($k$, $a_4$, and $a_6$) to achieve a target spherical aberration of 0.2 $\mu$m.

An IOL 100 having the above-described anterior-biased optical design (e.g., an IOL 100 having shape factors as depicted in FIG. 3 and anterior and posterior radii of curvature as depicted in FIG. 4) may reduce the prevalence of "glint," a phenomenon in which an external observer sees a reflection from an IOL implanted in a patient's eye. Human eye model simulations have shown that the intensity of the reflection depends on the anterior surface radius of curvature of the IOL, the intensity of the reflection being strongest in a certain range of radii of curvature that more closely match the curvature of the wavefront incident on the IOL (e.g., 18-40 mm). An IOL 100 having the above-described anterior-biased optical design may result in anterior surface radii of curvature across a broad range of lens powers (P) (e.g., lens powers (P) in the range of 12 to 35 diopters) that are outside the range known to cause the highest intensity reflections, thereby reducing the prevalence of glint.

Additionally, an IOL 100 having the above-described anterior-biased optical design (e.g., an IOL 100 having shape factors (X) corresponding to the curve depicted in FIG. 3 and anterior/posterior radii of curvature ($R_1/R_2$) corresponding to the curves depicted in FIG. 4) may result in a stable effective lens position (ELP) across a broad range of IOL powers due to the fact that the distance between the IOL principal plane and the IOL haptic plane ($\Delta_{PP}$, described below) remains substantially constant across the power range. This stability of ELP across the IOL power range may minimize A-constant variation, which may result in better and/or more predictable refractive outcomes

The above-described IOL haptic plane may be defined as a distance ($IOL_{HP}$) from an apex of the posterior surface as follows:

$$IOL_{HP} = Sag_{OE} + \frac{ET}{2} \qquad \text{Eq. (10)}$$

wherein,

$Sag_{OE}$ represents a distance between a posterior surface height at the apex of posterior surface 108 and a posterior surface height at the optic edge 112; and
ET represented the thickness at optic edge 112.

The above-described IOL principal plane may be defined as a distance ($IOL_{PP}$) from an apex of the posterior surface as follows:

$$IOL_{PP} = \frac{-n_1(IOL_{CT})(P_1)}{n_2(P)} \qquad \text{Eq. (11)}$$

wherein,

$n_1$ is a refractive index of aqueous humor or a patient's eye (approximately 1.336);
$n_2$ is a refractive index of the optic 102;
$IOL_{CT}$ is the center thickness of IOL 100;
$P_1$ is the anterior surface power; and
P is the IOL lens power.

6

Given Eq. (10) and Eq. (11), the distance between the IOL principal plane and the IOL haptic plane ($\Delta_{PP}$) can be defined as follows:

$$\Delta_{PP} = IOL_{HP} + IOL_{PP} = Sag_{OE} + \frac{ET}{2} - \frac{n_1(IOL_{CT})(P_1)}{n_1(P)} \qquad \text{Eq. (12)}$$

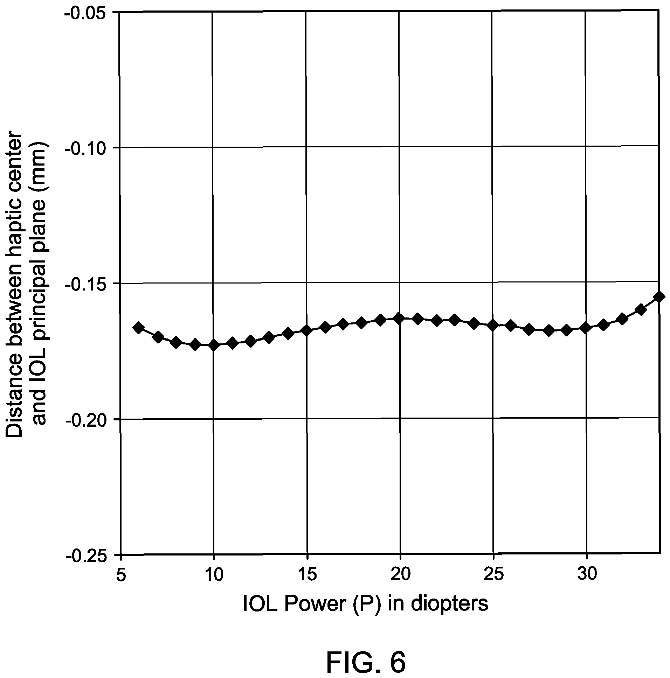

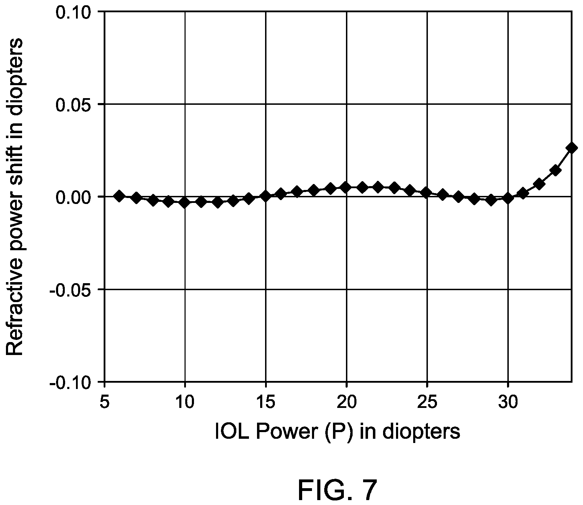

The above-described stability of effective lens position (ELP) is illustrated in FIG. 6, which depicts the distance between the IOL haptic plane and IOL principal plane ($\Delta_{PP}$) versus lens power (P) for an IOL 100 having shape factors (X) and anterior/posterior radii of curvature ($R_1/R_2$) as depicted in FIGS. 3 and 4 respectively. This is further illustrated by FIG. 7, which illustrates the power shift resulting from the distance between the IOL haptic plane and IOL principal plane ($\Delta_{PP}$) plotted in FIG. 6. Although FIGS. 6-7 illustrate $\Delta_{PP}$ and corresponding power shift for lenses having shape factors (X) and radii of curvature ($R_1/R_2$) equal to the curves depicted in FIGS 3-4, the present disclosure contemplates that, due to the above-described deviation of shape factors (X) and radii of curvature ($R_1/R_2$) from the curves depicted in FIGS 3-4 resulting from manufacturing constraints or other manufacturing considerations, there may be corresponding deviation from the curves depicted in FIGS. 6-7.

In embodiments in which there is deviation from the curve depicted in FIG. 6 for the reasons discussed above, the amount of acceptable variation in $\Delta_{PP}$ between any two lenses of different lens powers (P) may decrease with increased lens power (P). As just one example, the amount of acceptable variation at various lens powers (P) mat be as follows:

| IOL Power (D) | Range of acceptable variation (mm) |
| --- | --- |
| 6 | 0.29 |
| 10 | 0.17 |
| 20 | 0.08 |
| 30 | 0.05 |
| 34 | 0.04 |

Additionally, an IOL 100 having the above-described anterior-biased optical design (e.g., an IOL 100 having shape factors (X) corresponding to the curve depicted in FIG. 3 and anterior/posterior radii of curvature ($R_1/R_2$) corresponding to the curves depicted in FIG. 4) may be less sensitive to misalignment (e.g., decentration and tilt). More particularly, the above-described IOLs 100 have a positive shape factor, meaning they have a relatively high anterior surface curvature. The relatively high anterior surface curvature means that light rays incident on and outgoing from the anterior surface have a small average angle from normal directions such that the Gaussian optics formula deviates from Snell's law by a small amount, decreasing sensitivity to decentration. As a result, IOLs 100 having the above-described anterior-biased optical design may result in better refractive outcomes.

Finally, an IOL 100 having the above-described anterior-biased optical design (e.g., an IOL 100 having shape factors (X) as depicted in FIG. 3 and anterior/posterior radii of curvature ($R_1/R_2$) as depicted in FIG. 4) may reduce the incidence of negative dysphotopsia post-implantation. One reason for the reduction in the incidence of negative dysphotopsia is that the amount of posterior shift of the iris after cataract surgery may be reduced for IOLs having the above-described anterior-biased optical design. Because it is hypothesized that posterior iris shift may result in the patient experiencing negative dysphotopsia due to light hitting or missing different parts of the retina, reducing such shift may reduce the incidence of negative dysphotopsia. Another reason for the reduction in the incidence of negative dysphotopsia is that peripheral rays (rays entering the patient eye at a high incidence angle) that hit an IOL having the above-described anterior-biased optical design may be spread more evenly as compared to IOLs having an equi-convex design (i.e., same anterior and posterior curvatures). This even spreading of peripheral rays may reduce the perception of negative dysphotopsia.

## Claims

1.   A method of manufacturing an intraocular lens, IOL comprising an optic having an anterior surface and a posterior surface configured to be implanted in a patient's eye, wherein for each lens power (P) in a range of lens powers from 6 to 35 diopters, the method comprising:

selecting a curvature of the anterior surface, the anterior surface having an anterior surface radius of curvature ($R_1$) and an anterior surface power ($P_1$), the anterior surface power defined as:

$$P_1 = \frac{n_2 - n_1}{R_1};$$

selecting a curvature of the posterior surface, the posterior surface having a posterior surface radius of curvature $(R_2)$ and a posterior surface power $(P_2)$, the posterior surface power defined as:

$$P_2 = \frac{n_1 - n_2}{R_2};$$

wherein:

n$_1$ is a refractive index of aqueous humor of a patient's eye;
n$_2$ is a refractive index of the optic; and

selecting a shape factor (X), wherein the shape factor (X) is defined as:

$$X = \frac{R_2 - R_1}{R_2 + R_1};$$

wherein the shape factor (X) is greater than zero and corresponds to a curve defining shape factor (X) as a function of lens power (P), the curve monotonically decreasing with increased lens power (P), wherein the curve is defined by the following cubic equation: $X = X_0 + X_1 P + X_2 P^2 + X_3 P^3$;
wherein $X_0$, $X_1$, $X_2$, and $X_3$ are constants having values that are real numbers.

2. The method of claim 1, comprising defining the anterior surface power $(P_1)$ as a function of lens power (P) and shape factor (X).

3. The method of Claim 1, wherein the curve is non-linear.

4. The method of Claim 1, wherein the refractive index of the optic is in the range of 1.42 to 1.56.

5. The method of claim 1, wherein:

$X_0$ is in the range of 0.75 to 1.5;
$X_1$ is a negative number in the range of -0.11 to -0.05;
$X_2$, is in the range of 0.0017 to 0.0035; and
$X_3$ is in the range of -0.000042 to 0.00002.

6. The method of Claim 1, wherein:
the shape factor (X) is in the range of 0.20 to 1.0.

7. The method of Claim 1, further comprising providing a plurality of haptics extending from an edge of the optic and defining a haptic plane of the ophthalmic lens, wherein, for all lens powers (P) greater than 10 diopters, a distance between a principal plane of the optic and the haptic plane varies by an amount less than 0.2mm.

8. The method of Claim 1, further comprising providing a plurality of haptics extending from an edge of the optic and defining a haptic plane of the ophthalmic lens, wherein, for each lens having a lens powers (P) greater than 20 diopters, a distance between a principal plane of the optic and the haptic plane varies by an amount less than 0.1mm.

9. The method of claim 1, wherein:

$X_0$ is in the range of 0.75 to 1.5;
$X_1$ is a negative number in the range of -0.11 to -0.05;
$X_2$, is in the range of 0.0017 to 0.0035; and
$X_3$ is in the range of -0.000042 to 0.00002;

the shape factor (X) is in the range of 0.20 to 1.0 for lens powers (P) is in the range of 6 to 35 diopters; and

providing a plurality of haptics extending from the optic edge.

**10.** The method of Claim 9, wherein the anterior surface power $(P_1)$ is a function of lens power (P) and shape factor (X).

**11.** The method of claim 4 or the method Claim 9, wherein:

$X_0$ is approximately 1.068;
$X_1$ is approximately -0.075;
$X_2$, is approximately 0.0025; and
$X_3$ is approximately -0.00003.

**12.** The method of claim 1 or method of Claim 9, wherein the refractive index of the optic is approximately equal to 1.498.

**13.** The method of claim 1 or the method of Claim 9, wherein the anterior surface is an aspheric surface, and further comprising defining the sag of the aspheric surface as:

$$sag = \frac{cr^2}{1+\sqrt{1-(1+k)c^2r^2}} + a_4r^4 + a^6r^6 ;$$

r is a radial distance from the optical axis;
c is a base curvature of the anterior surface corresponding to the anterior surface radius of curvature $(R_1)$;
k is a conic constant; and
$a_4$ is a fourth order deformation constant; and

$a_6$ is a sixth order deformation constant and wherein k, a4, and a6 may be selected such that a target spherical aberration for an IOL is achieved.

**14.** The method of claim 1 or Claim 9, wherein, for each lens having a lens power (P) greater than 12 diopters, the anterior surface radius of curvature $(R_1)$ is less than 18mm.

**15.** The method of Claim 9, further comprising providing the plurality of haptics according to one or more of the following:

(i) wherein the plurality of haptics define a haptic plane of the intraocular lens; and for all lens powers (P) greater than 10 diopters, a distance between a principal plane of the optic and the haptic plane varies by an amount less than 0.2mm;
(ii) wherein the plurality of haptics define a haptic plane of the intraocular lens; and for all lens powers (P) greater than 20 diopters, a distance between a principal plane of the optic and the haptic plane varies by an amount less than 0.1mm.

**Patentansprüche**

**1.** Verfahren zum Herstellen einer Intraokularlinse, IOL, die eine Optik mit einer vorderen Oberfläche und einer hinteren Oberfläche umfasst, welche dazu konfiguriert ist, in ein Auge eines Patienten implantiert zu werden, wobei, für jede Linsenbrechkraft (P) in einem Bereich von Linsenbrechkräften von 6 bis 35 Dioptrien, das Verfahren Folgendes umfasst:

Auswählen einer Krümmung der vorderen Oberfläche, wobei die vordere Oberfläche einen vorderen Oberflächenkrümmungsradius $(R_1)$ und eine vordere Oberflächenbrechkraft $(P_1)$ aufweist, wobei die vordere Oberflächenbrechkraft definiert ist als:

$$P_1 = \frac{n_2-n_1}{R_1};$$

Auswählen einer Krümmung der hinteren Oberfläche, wobei die hintere Oberfläche einen hinteren Oberflächenkrümmungsradius $(R_2)$ und eine hintere Oberflächenbrechkraft $(P_2)$ aufweist, wobei die hintere Oberflächenbrechkraft definiert ist als:

$$P_2 = \frac{n_1 - n_2}{R_2};$$

wobei:

$n_1$ ein Brechungsindex von Kammerwasser eines Auges eines Patienten ist;
$n_2$ ein Brechungsindex der Optik ist; und
Auswählen eines Formfaktors (X), wobei der Formfaktor (X) definiert ist als:

$$X = \frac{R_2 - R_1}{R_2 + R_1};$$

wobei der Formfaktor (X) größer als null ist und einer Kurve entspricht, die einen Formfaktor (X) als eine Funktion der Linsenbrechkraft (P) definiert, wobei die Kurve mit zunehmender Linsenbrechkraft (P) monoton abnimmt, wobei die Kurve durch die folgende kubische Gleichung definiert ist: $X = X_0 + X_1 P + X_2 P^2 + X_3 P^3$;
wobei $X_0$, $X_1$, $X_2$ und $X_3$ Konstanten mit Werten sind, die reale Zahlen sind.

2. Verfahren nach Anspruch 1, das Definieren der vorderen Oberflächenbrechkraft ($P_1$) als eine Funktion einer Linsenbrechkraft (P) und eines Formfaktors (X) umfasst.

3. Verfahren nach Anspruch 1, wobei die Kurve nichtlinear ist.

4. Verfahren nach Anspruch 1, wobei der Brechungsindex der Optik in dem Bereich von 1,42 bis 1,56 liegt.

5. Verfahren nach Anspruch 1, wobei:

$X_0$ in dem Bereich von 0,75 bis 1,5 liegt;
$X_1$ eine negative Zahl in dem Bereich von -0,11 bis -0,05 ist;
$X_2$ in dem Bereich von 0,0017 bis 0,0035 liegt; und
$X_3$ in dem Bereich von -0,000042 bis 0,00002 liegt.

6. Verfahren nach Anspruch 1, wobei:
der Formfaktor (X) in dem Bereich von 0,20 bis 1,0 liegt.

7. Verfahren nach Anspruch 1, das ferner Bereitstellen mehrerer Haptiken umfasst, die sich von einem Rand der Optik erstrecken und eine Haptikebene der ophthalmischen Linse definieren, wobei für alle Linsenbrechkräfte (P) größer als 10 Dioptrien eine Entfernung zwischen einer Hauptebene der Optik und der Haptikebene um einen Betrag kleiner als 0,2 mm variiert.

8. Verfahren nach Anspruch 1, das ferner Bereitstellen mehrerer Haptiken umfasst, die sich von einem Rand der Optik erstrecken und eine Haptikebene der ophthalmischen Linse definieren, wobei für jede Linse mit einer Linsenbrechkraft (P) größer als 20 Dioptrien eine Entfernung zwischen einer Hauptebene der Optik und der Haptikebene um einen Betrag kleiner als 0,1 mm variiert.

9. Verfahren nach Anspruch 1, wobei:

$X_0$ in dem Bereich von 0,75 bis 1,5 liegt;
$X_1$ eine negative Zahl in dem Bereich von -0,11 bis -0,05 ist;
$X_2$ in dem Bereich von 0,0017 bis 0,0035 liegt; und
$X_3$ in dem Bereich von -0,000042 bis 0,00002 liegt;
der Formfaktor (X) in dem Bereich von 0,20 bis 1,0 für Linsenbrechkräfte (P) in dem Bereich von 6 bis 35 Dioptrien liegt; und
Bereitstellen mehrerer Haptiken, die sich von dem Optikrand erstrecken.

10. Verfahren nach Anspruch 9, die vordere Oberflächenbrechkraft ($P_1$) eine Funktion einer Linsenbrechkraft (P) und eines Formfaktors (X) ist.

**11.** Verfahren nach Anspruch 4 oder Verfahren nach Anspruch 9, wobei:

$X_0$ näherungsweise 1,068 ist;
$X_1$ näherungsweise -0,075 ist;
$X_2$ näherungsweise 0,0025 ist; und
$X_3$ näherungsweise -0,00003 ist.

**12.** Verfahren nach Anspruch 1 oder Verfahren nach Anspruch 9, wobei der Brechungsindex der Optik näherungsweise gleich 1,498 ist.

**13.** Verfahren nach Anspruch 1 oder Verfahren nach Anspruch 9, wobei die vordere Oberfläche eine asphärische Oberfläche ist, und welches ferner Definieren der Pfeilhöhe der asphärischen Oberfläche wie folgt umfasst:

$$Pfeilh\ddot{o}he = \frac{cr^2}{1 + \sqrt{1 - (1+k)c^2r^2}} + a_4 r^4 + a_6 r^6 \, ;$$

r eine radiale Entfernung von der optischen Achse ist;
c eine Basiskrümmung der vorderen Oberfläche entsprechend dem vorderen Oberflächenkrümmungsradius ($R_1$) ist;
k eine konische Konstante ist; und
$a_4$ eine Verformungskonstante vierter Ordnung ist; und
$a_6$ eine Verformungskonstante sechster Ordnung ist, und wobei k, a4 und a6 so gewählt werden können, dass eine sphärische Zielaberration für eine IOL erzielt wird.

**14.** Verfahren nach Anspruch 1 oder Anspruch 9, wobei für jede Linse mit einer Linsenbrechkraft (P) größer als 12 Dioptrien der vordere Oberflächenkrümmungsradius ($R_1$) kleiner als 18 mm ist.

**15.** Verfahren nach Anspruch 9, das ferner Bereitstellen der mehreren Haptiken gemäß einem oder mehreren des Folgenden umfasst:

(i) wobei die mehreren Haptiken eine Haptikebene der Intraokularlinse definieren; und, für alle Linsenbrechkräfte (P) größer als 10 Dioptrien, eine Entfernung zwischen einer Hauptebene der Optik und der Haptikebene um einen Betrag kleiner als 0,2 mm variiert;
(ii) wobei die mehreren Haptiken eine Haptikebene der Intraokularlinse definieren; und, für alle Linsenbrechkräfte (P) größer als 20 Dioptrien, eine Entfernung zwischen einer Hauptebene der Optik und der Haptikebene um einen Betrag kleiner als 0,1 mm variiert.

## Revendications

**1.** Procédé de fabrication d'une lentille intraoculaire, IOL, comprenant une optique ayant une surface antérieure et une surface postérieure configurée pour être implantée dans l'œil d'un patient, pour chaque puissance de lentille (P) dans une plage de puissances de lentilles de 6 à 35 dioptries, le procédé comprenant :

la sélection d'une courbure de la surface antérieure, la surface antérieure ayant un rayon de courbure de surface antérieure ($R_1$) et une puissance de surface antérieure ($P_1$), la puissance de surface antérieure étant définie comme suit :

$$P_1 = \frac{n_2 - n_1}{R_1};$$

la sélection d'une courbure de la surface postérieure, la surface postérieure ayant un rayon de courbure de surface postérieure ($R_2$) et une puissance de surface postérieure ($P_2$), la puissance de surface postérieure étant définie comme suit :

$$P_2 = \frac{n_1 - n_2}{R_2};$$

avec :

n$_1$ étant un indice de réfraction de l'humeur aqueuse de l'œil d'un patient ;
n$_2$ étant un indice de réfraction de l'optique ; et
la sélection d'un facteur de forme (X), le facteur de forme (X) étant défini comme suit :

$$X = \frac{R_2 - R_1}{R_2 + R_1};$$

le facteur de forme (X) étant supérieur à zéro et correspondant à une courbe définissant le facteur de forme (X) en fonction de la puissance de lentille (P), la courbe diminuant de façon monotone avec l'augmentation de la puissance de lentille (P), la courbe étant définie par l'équation cubique suivante : $X = X_0 + X_1 P + X_2 P^2 + X_3 P^3$ ;
avec $X_0$, $X_1$, $X_2$, et $X_3$ étant des constantes dont les valeurs sont des nombres réels.

2. Procédé selon la revendication 1, comprenant la définition de la puissance de surface antérieure (P$_1$) en fonction de la puissance de lentille (P) et du facteur de forme (X).

3. Procédé selon la revendication 1, la courbe étant non linéaire.

4. Procédé selon la revendication 1, l'indice de réfraction de l'optique étant dans la plage de 1,42 à 1,56.

5. Procédé selon la revendication 1,

   $X_0$ étant dans la plage de 0,75 à 1,5 ;
   $X_1$ étant un nombre négatif dans la plage de -0,11 à - 0,05 ;
   $X_2$ étant dans la plage de 0,0017 à 0,0035 ; et
   $X_3$ étant dans la plage de -0,000042 à 0,00002.

6. Procédé selon la revendication 1,
   le facteur de forme (X) étant dans la plage de 0,20 à 1,0.

7. Procédé selon la revendication 1, comprenant en outre la fourniture d'une pluralité d'haptiques s'étendant à partir d'un bord de l'optique et définissant un plan haptique de la lentille ophtalmique, pour toutes les puissances de lentilles (P) supérieures à 10 dioptries, une distance entre un plan principal de l'optique et le plan haptique variant d'une quantité inférieure à 0,2 mm.

8. Procédé selon la revendication 1, comprenant en outre la fourniture d'une pluralité d'haptiques s'étendant à partir d'un bord de l'optique et définissant un plan haptique de la lentille ophtalmique, pour chaque lentille ayant une puissance de lentille (P) supérieure à 20 dioptries, une distance entre un plan principal de l'optique et le plan haptique variant d'une quantité inférieure à 0,1 mm.

9. Procédé selon la revendication 1,

   $X_0$ étant dans la plage de 0,75 à 1,5 ;
   $X_1$ étant un nombre négatif dans la plage de -0,11 à - 0,05 ;
   $X_2$ étant dans la plage de 0,0017 à 0,0035 ; et
   $X_3$ étant dans la plage de -0,000042 à 0,00002 ;
   le facteur de forme (X) étant dans la plage de 0,20 à 1,0 pour des puissances de lentilles (P) comprises dans la plage de 6 à 35 dioptries ; et
   la fourniture d'une pluralité d'haptiques s'étendant à partir du bord optique.

10. Procédé selon la revendication 9, la puissance de surface antérieure (P$_1$) étant une fonction de la puissance de lentille (P) et du facteur de forme (X).

**11.** Procédé selon la revendication 4 ou procédé selon la revendication 9,

$X_0$ étant d'environ 1,068 ;
$X_1$ étant d'environ -0,075 ;
$X_2$, étant d'environ 0,0025 ; et
$X_3$ étant d'environ -0,00003.

**12.** Procédé selon la revendication 1 ou procédé selon la revendication 9, l'indice de réfraction de l'optique étant approximativement égal à 1,498.

**13.** Procédé selon la revendication 1 ou procédé selon la revendication 9, la surface antérieure étant une surface asphérique, et comprenant en outre la définition de la flèche de la surface asphérique comme :

$$sag = \frac{cr^2}{1 + \sqrt{1 - (1+k)c^2 r^2}} + a_4 r^4 + a^6 r^6 \; ;$$

r étant une distance radiale par rapport à l'axe optique ;
c étant une courbure de base de la surface antérieure correspondant au rayon de courbure de surface antérieure $(R_1)$ ;
k étant une constante de conicité ; et
$a_4$ étant une constante de déformation de quatrième ordre ; et
$a_6$ étant une constante de déformation de sixième ordre, k, a4 et a6 pouvant être sélectionnées de manière à obtenir une aberration sphérique cible pour une IOL.

**14.** Procédé selon la revendication 1 ou selon la revendication 9, pour chaque lentille ayant une puissance de lentille (P) supérieure à 12 dioptries, le rayon de courbure de surface antérieure $(R_1)$ étant inférieur à 18 mm.

**15.** Procédé selon la revendication 9, comprenant en outre la fourniture de la pluralité d'haptiques selon un ou plusieurs des éléments suivants :

(i) la pluralité d'haptiques définissant un plan haptique de la lentille intraoculaire ; et pour toutes les puissances de lentilles (P) supérieures à 10 dioptries, une distance entre un plan principal de l'optique et le plan haptique variant d'une quantité inférieure à 0,2 mm ;
(ii) la pluralité d'haptiques définissant un plan haptique de la lentille intraoculaire ; et pour toutes les puissances de lentilles (P) supérieures à 20 dioptries, une distance entre un plan principal de l'optique et le plan haptique variant d'une quantité inférieure à 0,1 mm.

FIG. 1

FIG. 2

FIG. 3

FIG. 4

FIG. 5

FIG. 6

FIG. 7

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2015320547 A
- US 2017112612 A
- US 2007093891 A
- US 2006227286 A

- US 5290892 A
- US 5693095 A
- US 8449610 B
- US 8969429 B